Europäisches Patentamt

European Patent Office

Office européen des brevets

(19)

(11) Numéro de publication: **0 147 492**
**A1**

## (12) DEMANDE DE BREVET EUROPEEN

(21) Numéro de dépôt: 83402564.5

(22) Date de dépôt: 30.12.83

(51) Int. Cl.⁴: **A 61 B 5/10**

(43) Date de publication de la demande:
10.07.85 Bulletin 85/28

(84) Etats contractants désignés:
AT BE CH DE GB IT LI LU NL SE

(71) Demandeur: Chanchole, Serge Félix Roger
89 bis rue de la Division Leclerc
F-91310 Linas (Essonne)(FR)

(71) Demandeur: Lepetit, Jean-Marc
32 avenue de la Liberation
F-87000 Limoges (Haute-Vienne)(FR)

(72) Inventeur: Chanchole, Serge Félix Roger
89 bis rue de la Division Leclerc
F-91310 Linas (Essonne)(FR)

(72) Inventeur: Lepetit, Jean-Marc
32 avenue de la Liberation
F-87000 Limoges (Haute-Vienne)(FR)

(74) Mandataire: Chevallier, Robert Marie Georges
Cabinet BOETTCHER 23, rue La Boétie
F-75008 Paris(FR)

(54) **Instrument médical à ultrasons notamment pour l'analyse du pouls cérébral.**

(57) L'appareil comprend une sonde (1) fonctionnant en émetteur-récepteur, un générateur (2) de train d'impulsions d'excitation alimentant la sonde (1), la sortie de la sonde (1) étant reliée à un amplificateur (3) à correction de gain réglable (6) suivant une évolution prédéterminée, un dispositif d'extraction (7) d'une suite d'échos reçus par la sonde (1) et correspondant à une impulsion d'excitation émise par la sonde (1), un dispositif de filtrage ($9_1$ - $9_N$) comportant des canaux à filtre associés à un certain nombre d'échos séparés par le dispositif d'extraction (7) et un circuit d'exploitation (10, 11, 12, 13, 14, 15, 16) des signaux recueillis.

Fig. 1

EP 0 147 492 A1

Instrument médical à ultrasons notamment pour l'analyse
du pouls cérébral.

La présente invention concerne un instrument
médical à ultrasons notamment pour l'analyse du pouls cérébral
à diverses profondeurs à partir de la paroi crânienne à
l'aide de faisceaux d'ultrasons réfléchis sur les structures
internes et essentiellement les vaisseaux sanguins du cerveau.

Un tel dispositif permet d'estimer l'état fonctionnel des tissus organiques du cerveau, de visualiser l'activité circulatoire du cerveau sur un écran tel qu'un tube
cathodique de télévision.

On connaît déjà diverses techniques d'examen du
cerveau :

La technique la plus ancienne est l'électroencéphalographie qui consiste à mesurer les variations de
potentiel électrique du cerveau à l'aide d'électrodes placées
sur le cuir chevelu.

La radiologie avec les scanners à rayons X en ce
qui concerne la mesure tomo-densitométrique et les procédés
artériographiques pour visualiser l'arbre vasculaire.

La médecine nucléaire par fixation sélective
d'isotopes avec de nouvelles méthodes telles que la tomographie par émission de positons.

Les procédés à ultrasons selon les techniques A, B
et Doppler.

Une technique récente de résonance magnétique
nucléaire qui est très délicate à mettre en oeuvre à cause
de la nécessité d'une parfaite constance du champ de référence.

De façon générale, les méthodes les plus actuelles
- essentiellement dans le domaine de la médecine nucléaire -
sont lourdes, difficiles à mettre en oeuvre, coûteuses et
souvent dangereuses pour le patient.

Les techniques classiques telles que l'électroencéphalographie ont leurs limites maintenant bien connues :
pour cette dernière, la difficulté de rattacher le phénomène
analysé à des paramètres objectifs.

L'ultrasonographie Doppler ne permet qu'une appréciation globale et non zonale des phénomènes circulatoires.

Il convient de remarquer que le comportement sphygmographique ultrasonore zonal du cerveau dépend d'un certain nombre d'éléments physiologiques, à savoir :

1 - la qualité de la pompe cardiaque,

2 - la qualité de l'arbre vasculaire,

3 - la qualité du contenu vasculaire,

4 - Les variations qualitatives des résistances vasculaires sous diverses influences,

5 - Les variations quantitatives du débit sanguin cérébral,

6 - Les modifications de la pression intra-crânienne,

7 - Les modifications de la densité tissulaire cérébrale et les modifications de son élasticité.

La présente invention a pour but de créer un instrument médical permettant d'apprécier de façon simple, rapide, peu coûteuse ainsi que répétitive à volonté, car sans danger pour le patient, le caractère fonctionnel de zones tissulaires déterminées, par exemple l'élasticité des artères et la réponse du lit vasculaire à une stimulation interne ou externe telle qu'une absorption de drogue, une inhalation de gaz, etc.. en permettant l'examen du comportement sphygmographique ultrasonore zonal du cerveau par l'examen des réponses élémentaires des différentes zones à une excitation ultrasonore et le traitement par combinaison ou association, comparaison, etc... des diverses réponses élémentaires.

A cet effet, l'invention concerne un instrument médical à ultrasons notamment pour l'analyse du pouls cérébral à partir de la paroi crânienne par des faisceaux d'ultrasons émis par un émetteur d'ultrasons, instrument médical caractérisé en ce qu'il comporte au moins une sonde fonctionnant en émetteur-récepteur, un générateur de train d'impulsions d'excitation alimentant la sonde, la sortie de la sonde étant reliée à un amplificateur à correction de gain réglable suivant une évolution prédéterminée, un dispositif d'extraction d'une suite d'échos reçus par la sonde et correspondant à une impulsion d'excitation émise par la sonde, un dispositif de filtrage pour différents canaux associés à un certain nombre d'échos séparés par le dispositif d'extraction et un circuit d'exploitation des signaux recueillis.

L'instrument médical permet ainsi de recueillir une suite d'échos associés à des dioptres ultrasonores dont on veut analyser l'état, par exemple par comparaison avec des grandeurs ou des courbes de référence. En recueillant une succession d'échos homologues, on peut, par intégration, obtenir une courbe traduisant l'évolution de chaque écho dans le temps. La séparation des échos dans le dispositif d'extraction se fait par un ensemble de fenêtres réglables ou non, juxtaposés ou non, permettant d'isoler les échos intéressants ; cette séparation est d'autant plus facile que les échos auront été préalablement amplifiés pour compenser leur atténuation.

En effet, suivant une autre caractéristique, l'instrument médical comporte un circuit de correction de gain relié à l'entrée de commande de gain de l'amplificateur, ce circuit de correction de gain étant synchronisé par le générateur de synchronisation pour assurer une évolution réglable du gain de l'amplificateur en fonction du temps et/ou d'un autre paramètre physique.

Pour obtenir une évolution des signaux dans le temps, il est prévu de relier le circuit d'extraction à un circuit de cyclage automatique ; ce circuit commande le balayage automatique de la suite d'échos fournis au circuit d'extraction par la sonde et l'amplificateur en réponse à chaque impulsion d'excitation émise par la sonde, pour séparer les différents échos.

Suivant une autre caractéristique, le circuit d'exploitation comporte un convertisseur analogique/ numérique recevant les signaux de sortie des différents canaux du dispositif de filtrage pour fournir les signaux numériques ainsi obtenus à un calculateur commandant un dispositif d'affichage (écran cathodique) et/ou une imprimante.

Pour faciliter les mesures et permettre l'analyse des deux hémisphères cérébraux, il est avantageux que l'instrument comporte au moins deux ensembles parallèles formés chacun d'une sonde, d'un amplificateur, d'un circuit d'extraction et de circuits de filtrage correspondants pour permettre une double analyse simultanée.

La présente invention sera décrite plus en détail à l'aide des dessins annexés dans lesquels :

- la figure 1 est un schéma d'ensemble de l'instrument médical, selon l'invention,

- les figures 2A-2I' sont des chronogrammes de différents signaux obtenus dans l'instrument selon la figure 1,

- les figures 3A, 3B représentent l'évolution d'un signal écho en fonction du temps.

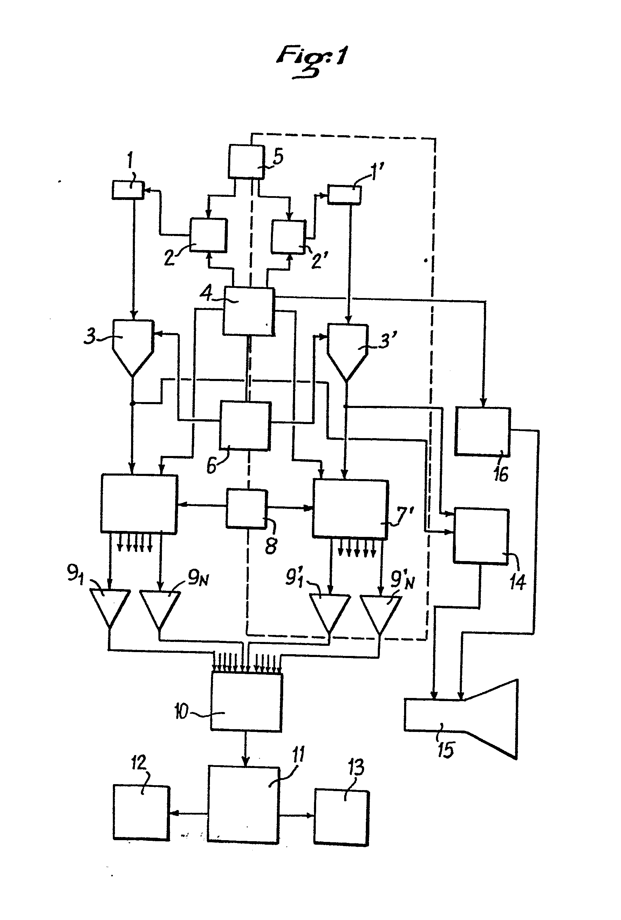

Selon la figure 1, l'instrument médical à ultrasons pour l'analyse du pouls cérébral à différentes profondeurs à partir de la paroi crânienne se compose d'une sonde 1 fonctionnant à la fois comme émetteur et comme récepteur. La sonde 1 reçoit des signaux impulsionnels d'excitation fournis par le générateur 2, lui-même alimenté à partir du circuit d'alimentation à haute tension 5. Les signaux fournis à la sonde 1 par le générateur 2 se traduisent par l'émission de signaux ultrasonores, la sonde 1 fonctionnant comme émetteur. En retour, la sonde 1 reçoit les signaux de réponse ou suite d'échos renvoyés par les différentes zones du volume analysé par la sonde 1.

Les signaux d'écho sont fournis à un amplificateur 3 dont le gain est réglé dans le temps par un circuit de correction de gain 6 de façon à tenir compte de

5

l'atténuation du signal émis par la seconde 1 et de la suite des échos reçus par la sonde 1 en fonction de l'éloignement des surfaces à détecter, ou de l'atténuation différente des ondes par les différentes zones du volume analysé.

La sortie de l'amplificateur 3 est reliée à un circuit d'extraction de signaux 7 à N sorties reliées chacune à un filtre $9_1$-$9_N$. La synchronisation entre le générateur d'impulsions d'émission 2 et le circuit d'extraction de signaux d'écho 7 est assurée par un générateur de synchronisation 4.

Le circuit d'extraction 7 comporte autant de sorties qu'il y a de signaux à séparer dans la suite des signaux d'écho. Ce nombre peut être surdimensionné et certains canaux peuvent être interdits suivant les applications. Le circuit 7 fournit ainsi des signaux correspondant à chaque écho particulier, intéressant, pour le circuit d'exploitation éventuellement après conversion analogique/numérique, par affichage sur un écran cathodique ou impression par une imprimante ou une table traçante.

De façon plus détaillée, la sonde 1 est un émetteur-récepteur d'ultrasons ; cette sonde est de préférence réalisée en zirconate-titanate de plomb et travaille à une fréquence comprise entre 1,5 MHZ et 2,5 MHZ. Cette sonde est placée de préférence contre une paroi latérale de la boîte crânienne d'un patient puisque des considérations physico-médicales montrent qu'il est difficile d'explorer l'ensemble du cerveau à l'aide d'une seule sonde et qu'il est intéressant de limiter l'exploration à un seul hémisphère par sonde.

Le générateur d'impulsions 2 fournit des impulsions de tension relativement élevées de l'ordre de 700 volts à la sonde 1 qui fonctionne alors comme émettrice. En fait, les impulsions fournies par le générateur 2 sont suffisamment espacées pour que la sonde 1 puisse d'abord émettre une impulsion dans la boîte crânienne, puis recevoir les signaux d'écho de cette impulsion renvoyés par les différents dioptres ultrasonores à l'intérieur du cerveau. De façon générale, les dioptres ultrasonores du cerveau sont les parois des vaisseaux sanguins, les membranes, etc.. ; la variation des caractéristiques de réflexion de ces dioptres sous l'effet de la poussée systolique engendre des échos qui varient dans le temps.

Après l'émission d'une impulsion, la sonde 1 est excitée par les signaux d'écho renvoyés par la boîte crânienne en réponse à cette impulsion d'entrée. Ces échos sont transformés en signaux électriques par la sonde qui les envoie à l'amplificateur 3.

La succession des signaux d'impulsion fournis par le générateur 2 est telle qu'elle permette la réception en retour de la suite des échos ou du moins le retour des échos les plus intéressants. Comme déjà indiqué, l'analyse aux ultrasons appliqués au pouls cérébral ne peut intéresser valablement qu'un hémisphère cérébral, l'hémisphère opposé ne renvoyant que des échos très atténués et difficilement exploitables.

Pour tenir compte d'une part, de l'atténuation du signal impulsionnel émis par la sonde 1 au fur et à mesure de sa progression à l'intérieur du cerveau et d'autre part, de l'atténuation des signaux d'écho renvoyés par les différents dioptres du cerveau en réponse au signal impulsionnel d'entrée, sur le chemin du retour, le gain de l'amplificateur 3 est commandé dans le temps par un circuit de correction de gain 6 qui donne une correction sensiblement logarithmique du gain puisque l'atténuation des signaux dans le cerveau est une fonction exponentielle de la distance séparant le point de propagation de l'onde et le point d'entrée de l'onde dans le cerveau.

De façon simple, le gain de l'amplificateur 3, commandé par le circuit de correction de gain 6, évolue de façon croissante au cours d'un cycle, c'est-à-dire pour une suite d'échos issus d'une même impulsion d'excitation. Toutefois, une approche plus fine peut être envisagée si l'atténuation du volume analysé devait ne pas varier de façon relativement continue en fonction de l'éloignement par rapport à la sonde. La courbe du gain se règle sur le correcteur de gain 6 essentiellement en fonction de l'expérience recueillie au cours des essais.

Ce circuit de correction de gain 6 est également relié au générateur de synchronisation 4 pour positionner correctement la courbe du gain en fonction de l'état de fonctionnement de la sonde 1 et de l'amplificateur 3.

Le circuit d'extraction 7 est en quelque sorte un convertisseur série-parallèle qui reçoit la suite des signaux d'échos après amplification par l'amplificateur 3 pour séparer ces échos en des signaux parallèles fournis aux sorties respectives.

Le circuit d'extraction 7 comporte N fenêtres associées à chaque sortie et qui se positionnent dans le temps et ont une largeur réglable ou non. Les différentes fenêtres du circuit d'extraction 7 sont positionnées en fonction de la position des échos dans la suite des échos d'un cycle de fonctionnement.

En effet, la position des échos dans un signal de réponse à une impulsion d'excitation émise par la sonde 1 change peu, la position de la sonde 1 restant fixe et c'est surtout l'amplitude des échos qui varie dans le temps en fonction de différents paramètres physiologiques. Les différents canaux de sortie du circuit d'extraction 7 sont reliés chacun à un filtre $9_1 - 0_N$ qui assure l'intégration des impulsions respectives pour fournir une courbe continue traduisant l'évolution de chaque impulsion en fonction du temps.

Les signaux analogiques ainsi fournis par les différents filtres $9_1 - 9_N$ sont transmis à un convertisseur analogique/numérique 10 qui transforme ces signaux en des signaux numériques susceptibles d'être exploités par un calculateur numérique 11 qui alimente un écran cathodique 12, une imprimante ou table traçante 13 ou encore enregistre ces signaux dans une mémoire non représentée ou les fournit à un système de traitement plus puissant.

Il est également possible de surveiller l'évolution des signaux de réponse en sortie d'amplificateur 3 à l'aide d'un tube cathodique 15 dont le circuit de balayage horizontal 16 est relié au générateur de synchronisation 4, le tube cathodique 15 étant également attaqué par un amplificateur de balayage vertical 14 qui est commandé par le signal de sortie de l'amplificateur 3.

Bien que de façon générale, l'instrument médical selon l'invention puisse se suffire d'une seule sonde 1, il est préférable pour faciliter le diagnostic et le rendre plus rapide, de dédoubler au moins une partie des moyens de cet instrument médical et d'avoir une seconde sonde 1', ainsi que des éléments symétriques à ceux du circuit d'exploitation de la sonde 1, c'est-à-dire un générateur de signaux impulsionnels 2', un amplificateur 3' qui reçoit les signaux de sortie de la sonde 1', un circuit d'extraction des signaux d'écho 7' ainsi que des filtres $9'_1$-$9'_N$. Les moyens tels que l'alimentation haute tension 5, le générateur de synchronisation 4, le circuit de correction de gain 6 ainsi que le tube cathodique 15, le circuit de balayage horizontal 16, l'amplificateur de balayage vertical 14, peuvent être communs aux deux branches de cet instrument médical.

Dans le cas de deux sondes 1, 1', il suffit de choisir un convertisseur analogique/numérique 10 dont le nombre d'entrées est égal au nombre de sorties des circuits d'extraction 7, 7'. En aval du convertisseur analogique/numérique 10, le calculateur 11, l'écran cathodique 12, l'imprimante 13 et les éventuels périphériques peuvent être communs aux deux branches de l'instrument médical.

Le fonctionnement du circuit d'extraction des signaux d'écho est synchronisé par le générateur de synchronisation 4 ; de plus, ce circuit est également relié à un circuit de fonctionnement cyclique automatique 8 qui assure la mise en oeuvre successive et répétée des fenêtres des différents canaux donnant les signaux de sortie alimentant les figures $9_1-9_N$.

Le fonctionnement de l'instrument médical, représenté schématiquement à la figure 1 ainsi que l'exploitation des signaux fournis par ces instruments seront explicités à l'aide des figures 2A-2I' et 3A-3B.

Dans les différentes figures 2A-3B, les ordonnées représentent des tensions et les abscisses le temps.

La figure 2A représente les impulsions de cadencement général fournies par une horloge interne du générateur de synchronisation 4. Le générateur de synchronisation 4 fournit des impulsions d'excitation correspondant à la courbe de la figure 2B pour la sonde 1. Les impulsions d'excitation fournies à la sonde 1' sont données par la courbe de la figure 2B'. Selon le mode de réalisation décrit, une impulsion de cadencement sur deux est associée au fonctionnement de la sonde 1 et l'impulsion de cadencement intermédiaire est associée au fonctionnement de la sonde 1'.

La figure 2C montre la suite des signaux d'écho $S_1$, $S_2$, $Sm_1$, $Sm$, reçus par la sonde 1 en réponse au signal impulsionnel émis. Le signal de réponse est constitué par un train d'impulsions correspondant aux différents dioptres rencontrés par le signal impulsionnel d'entrée.

A chaque impulsion d'entrée selon la figure 2B correspond un train d'impulsions de réponse.

La figure 2C' représente le signal de réponse au signal impulsionnel selon la courbe 2B'. Ces trains d'impulsions sont analogues entre eux et ne diffèrent en principe que par l'amplitude de leurs impulsions.

Le circuit de correction de gain 6, commandé par le générateur de synchronisation 4, commande l'évolution du gain de l'amplificateur 3 ou de l'amplificateur 3', suivant une courbe représentée à la figure 2D. La courbe du gain selon la figure 2D est répétée pour chaque impulsion de cadencement fournie par le générateur 4 puisque cette courbe de correction de gain est destinée à la fois aux signaux fournis par la sonde 1 et aux signaux fournis par la sonde 1'.

Après correction du gain de l'amplificateur 3, 3', puis redressement on obtient le signal d'écho représenté aux figures 2E, 2E'.

Le circuit d'extraction des signaux d'écho 7, synchronisé par le générateur de synchronisation 4, fournit à ses différentes sorties des signaux représentant chacun un écho de la suite d'échos de la figure 2E. Les différents signaux recueillis ainsi sur les différentes sorties du circuit d'extraction 7 sont représentées aux courbes des figures 2F, 2G, 2H, 2I. Dans l'intervalle de fonctionnement correspondant, le circuit d'extraction 7' agit comme le circuit d'extraction 7 et fournit les signaux représentés aux courbes 2F', 2H', 2I'.

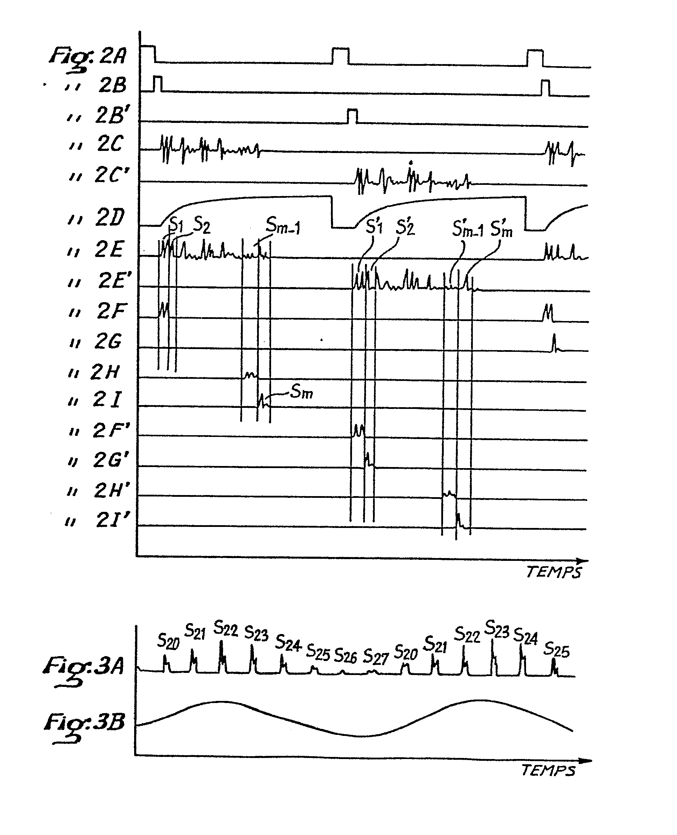

Les figures 3A, 3B montrent l'évolution de l'amplitude d'un signal, par exemple du signal correspondant à la seconde sortie (figure 2G) en fonction du temps. La figure 3A montre la succession des signaux d'échos $S_{20}$, $S_{21}$...$S_{27}$ obtenus au cours d'un certain nombre de cycles successifs (pour une même sonde). Le filtre $9_2$, associé à ce signal, en assure l'intégration et fournit en sortie un signal continu représenté à la figure 3B. L'examen de ce signal et sa comparaison avec des signaux de référence permet de tirer des conclusions médicales.

En tenant compte des considérations développées sommairement dans le préambule de la présente demande,

11

on peut définir dans le cas d'une analyse cérébrale, un index sphygmométrique. Cet index est une fonction associative de l'amplitude et de la morphologie (spectre de fréquence) des courbes zonales du pouls cérébral.

On peut ainsi définir les index sphygmométriques suivants à partir des zones d'investigation d'un cm de profondeur :

A - L'index sphygmométrique carotidien global droit qui est la somme des amplitudes des pouls des zones 3, 4, 5, 6, 7 de l'hémisphère droit du cerveau, divisée par la somme totale des amplitudes du pouls dans toutes les zones des deux hémisphères.

B - L'index sphygmométrique carotidien global gauche qui est défini comme pour le point A mais pour l'hémisphère cérébral gauche.

C - L'index sphygmométrique cortical droit défini par le rapport de l'amplitude du pouls de la zone N° 3 de l'hémisphère droit à la somme totale des amplitudes du pouls dans toutes les zones des deux hémisphères.

D - L'index sphygmométrique cortical gauche défini comme ci-dessus mais pour l'hémisphère gauche.

E - L'index sphygmométrique sylvien superficiel droit défini par le rapport de la somme des amplitudes du pouls des zones 3, 4, 5 de l'hémisphère droit à la somme totale des amplitudes du pouls dans toutes les zones des deux hémisphères.

F - L'index sphygmométrique sylvien superficiel gauche défini comme ci-dessus mais pour l'hémisphère gauche.

Cette liste d'index sphygmométrique n'est pas limitative et on peut par exemple en procédant de façon analogue, définir des index sphygmométriques vertébro-basilaires.

L'invention permet le calcul automatique ou non des index sphygmométriques ainsi que leur affichage ou leur enregistrement étant donné la cohérence parfaite entre les variations de ces index et les constatations cliniques non détaillées dans la présente description.

L'invention permet également l'estimation des débits sanguins locaux et la mesure des pressions intracrâniennes et cela de façon externe. L'instrument selon l'invention permet également l'établissement d'un pronostic par l'étude des réactions du lit vasculaire à des stimuli ainsi que l'appréciation immédiate de l'effet d'une thérapie telle que l'ingestion de médicaments. Cela est particulièrement important dans le cas de la réanimation et de soins intensifs. De plus, l'instrument médical est léger et peut être placé près du patient (sans nécessiter le déplacement du patient ce qui est impossible dans de nombreux cas).

L'instrument médical selon l'invention peut comporter également un dispositif pour permettre d'extraire, soit par voie analogique, soit par voie numérique, la valeur de la pression intra-crânienne ou encore des moyens analogiques ou numériques permettant de déterminer la valeur d'un débit sanguin local.

Enfin, l'invention permet de déterminer l'état de mort cérébral par l'absence de circulation sanguine cérébrale. Comme indiqué ci-dessus, les différents examens se font en général pour un hémisphère cérébral (gauche ou droit), une analyse analogue s'effectuant sur l'autre moitié. Selon la variante de réalisation de l'invention comportant au moins deux sondes, on effectue de façon précise simultanée l'analyse de chaque moitié du cerveau. Cette analyse simultanée des deux moitiés permet des conclusions par comparaison des signaux.

Enfin, et de façon générale, les informations ainsi recueillies par analyse d'une suite d'échos en réaction à une impulsion d'excitation, peut se faire suivant des programmes extrêmement sophistiqués, soit à l'aide d'un petit ordinateur, soit à l'aide d'un système plus puissant, par exemple à des fins statistiques, etc..

## REVENDICATIONS

1. Instrument médical à ultrasons notamment pour l'analyse du pouls cérébral à partir de la paroi crânienne par des faisceaux d'ultrasons, émis par un émetteur d'ultrasons, instrument médical caractérisé en ce qu'il comporte au moins une sonde (1) fonctionnant en émetteur-récepteur, un générateur (2) de train d'impulsions d'excitation alimentant la sonde (1), la sortie de la sonde (1) étant reliée à un amplificateur (3) à correction de gain réglable (6) suivant une évolution prédéterminée, un dispositif d'extraction (7) d'une suite d'échos reçus par la sonde (1) et correspondant à une impulsion d'excitation émise par la sonde (1), un dispositif de filtrage ($9_1$ -$9_N$) comportant des canaux à filtre associés à un certain nombre d'échos séparés par le dispositif d'extraction (7) et un circuit d'exploitation ( 10, 11, 12, 13, 14, 15, 16) des signaux recueillis.

2. Instrument médical selon la revendication 1, caractérisé en ce que le circuit d'extraction (7) est relié à un circuit de cyclage automatique (8) lui-même relié au générateur de synchronisation (4) pour commander le balayage automatique de la suite d'échos fournis au circuit d'extraction (7) par la sonde (1) et l'amplificateur (3) en réponse à chaque impulsion d'excitation émise par la sonde (1), pour séparer les échos correspondant aux différentes couches à examiner.

3. Instrument médical selon la revendication 1, caractérisé en ce que le circuit d'exploitation comporte un convertisseur analogique/numérique (10) recevant les signaux de sortie des différents canaux du dispositif de filtrage ($9_1$-$9_N$), pour fournir les signaux numériques ainsi obtenus à un calculateur (11) commandant un dispositif d'affichage (12) (écran cathodique) et/ou une imprimante (13).

4. Instrument médical selon la revendication 1, caractérisé en ce que le circuit d'exploitation comporte un tube cathodique (15), un circuit de balayage horizontal (16) relié au générateur de synchronisation (4) et un circuit de balayage vertical (14) relié à la sonde (1) (par l'intermédiaire de l'amplificateur (3).

5. Instrument médical selon l'une quelconque des revendications 1 à 4, caractérisé en ce qu'il comporte au moins deux ensembles parallèles formés chacun d'une sonde (1, 1') d'un amplificateur (3, 3'), d'un circuit d'extraction (7,7') et de circuits de filtrage ($9_1 - 9_N$ et $9'_1 - 9'_N$) correspondants pour permettre une double analyse simultanée.

6. Instrument médical à ultrasons selon l'une quelconque des revendications 1 à 5, caractérisé en ce qu'il comporte un dispositif permettant d'extraire soit par un analogique, soit par un moyen numérique la valeur de la pression intra-crânienne.

7. Instrument médical selon l'une quelconque des revendications 1 à 6, caractérisé en ce qu'il comporte un dispositif permettant d'extraire soit analogiquement, soit numériquement la valeur d'un débit sanguin local.

*Fig:1*

Fig. 2A

,, 2B

,, 2B'

,, 2C

,, 2C'

,, 2D

,, 2E

,, 2E'

,, 2F

,, 2G

,, 2H

,, 2I

,, 2F'

,, 2G'

,, 2H'

,, 2I'

$S_1$ $S_2$   $S_{m-1}$     $S'_1$ $S'_2$   $S'_{m-1}$ $S'_m$

$S_m$

TEMPS

Fig. 3A

Fig. 3B

$S_{20}$ $S_{21}$ $S_{22}$ $S_{23}$ $S_{24}$ $S_{25}$ $S_{26}$ $S_{27}$ $S_{20}$ $S_{21}$ $S_{22}$ $S_{23}$ $S_{24}$ $S_{25}$

TEMPS

Office européen
des brevets

**RAPPORT DE RECHERCHE EUROPEENNE**

EP 83 40 2564

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | ULTRASONICS, vol. 8, no. 2, avril 1970, Guildford (GB); D.E. ROBINSON et al.:"The CAL four channel solid state echo-encephaloscope", pages 93-96 <br> * Page 94, colonne de gauche, ligne 11 - colonne de droite, ligne 19; figure 5 * | 1,2 | A 61 B  5/10 |
| | --- | | |
| X | FR-A-1 471 475 (AUTOMATION INDUSTRIES, INC.) <br> * Page 3, colonne de gauche, lignes 18-20 * | 1,2 | |
| | --- | | |
| X | MEDICAL BIOLOGICAL ENGINEERING, vol. 9, no. 4, Pergamon Press, Stevenage, Herts (GB); J.M. CLARK et al.:"The measurement of intracranial echo pulsations", pages 263-287 <br> * Page 268, colonne de gauche, lignes 21-24, 36-40 * | 1,2 | DOMAINES TECHNIQUES RECHERCHES (Int. Cl ³) <br><br> A 61 B  10/00 <br> A 61 B   5/00 |
| | --- | | |
| A | IEEE TRANSACTIONS ON SONICS AND ULTRASONICS, vol. SU-29, no. 1, janvier 1982, New York (US); W.A. ERDMANN et al.:"Instrumentation for ultrasonic transkull visualization", pages 5-11 <br> * Page 7, figure 6 * | 3 | |
| | ----- | | |

Le présent rapport de recherche a été établi pour toutes les revendications

| Lieu de la recherche | Date d'achèvement de la recherche | Examinateur |
|---|---|---|
| LA HAYE | 08-08-1984 | ONILLON C.G.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82